Europäisches Patentamt

**European Patent Office**

**Office européen des brevets**

(11) Numéro de publication: **0 100 762**
**A1**

(12) **DEMANDE DE BREVET EUROPEEN**

(21) Numéro de dépôt: **83870054.0**

(22) Date de dépôt: **08.06.83**

(51) Int. Cl.³: **A 61 M 1/03**

(30) Priorité: **11.06.82 BE 208327**

(43) Date de publication de la demande:
**15.02.84 Bulletin 84/7**

(84) Etats contractants désignés:
**IT**

(71) Demandeur: **Ravet, Albert**
**108, rue du Crucifix**
**B-4400 Herstal(BE)**

(72) Inventeur: **Godon, Jean-Pierre, Dr.**
**Fays 129**
**B-4861 Soiron-Pepinster(BE)**

(72) Inventeur: **Simon, Théo**
**Rue d'Affnay, 33 Parc de Fayenbois sur**
**B-4610 Beyne-Heusay(BE)**

(74) Mandataire: **van Malderen, Michel et al,**
**p.a. FREYLINGER & ASSOCIES 85/042 Boulevard de la**
**Sauvenière**
**B-4000 Liège(BE)**

(54) Appareil pour hémodialyse sans anticoagulants.

(57) Appareil pour la pratique de l'hémodialyse sans anticoagulants, comportant un hémodialyseur (10) dont le tuyau d'entrée (1) sur lequel est montée une pompe volumétrique (9) est la réunion d'un conduit d'amenée (4) de sang prélevé au patient et d'un conduit d'alimentation (5) de liquide physiologique, chacun de ces conduits (4, 5) comportant un organe de contrôle de passage (7, 8) commandé pour ouvrir et fermer alternativement chaque conduit (4, 5).

FIG. 1

EP 0 100 762 A1

Appareil pour la pratique de l'hémodialyse sans
anticoagulants et utilisation de celui-ci.

La présente invention concerne un appareil permettant de pratiquer les hémodialyses sans avoir recours
à des anticoagulants et leurs antidotes; elle concerne
également l'utilisation dudit appareil.

La technique d'hémodialyse permet le traitement
de patients atteints d'insuffisance rénale aiguë et
chronique. En général, dans les appareillages connus,
le sang du patient passe dans un circuit extracorporel
et dans un "hémodialyseur" où le sang circule en contact
avec une membrane semi-perméable destinée à la dialyse
du sang. Cette membrane est généralement constituée
de cuprophane , de poly-acrylo-nitrile ou d'acétate
de cellulose. Etant donné que cette membrane est un
corps étranger pour le sang, celui-ci coagule après
10 à 20 minutes de contact, rendant ainsi la poursuite
du traitement impossible. Pour éviter cette coagulation,
il est nécessaire d'injecter dans le sang du patient
un anticoagulant, comme l'héparine. Si pour la plupart
des patients, ce traitement par anticoagulant ne présente
aucun risque, il n'en va pas de même pour les patients
souffrant d'une hypertension artérielle sévère avec
hémorragie du fond de l'oeil, les patients diabétiques,
les patients ayant subi un traumatisme, les patients
récemment opérés et les patients présentant des
hémorragies notamment digestives, qui représentent
jusqu'à environ 20 % des patients traités dans un
centre d'hémodialyse.

Jusqu'à présent, le risque d'hémorragie, chez
certains patients, a été réduit en administrant à
l'entrée de l'hémodialyseur une solution d'anticoagulant
en perfusion continue et, à la sortie de l'hémodialyseur,
l'antidote de celui-ci, à savoir l'héparine et son antidote, le sulfate de protamine. La précision de cette méthode est cependant très aléatoire. En effet, la dose d'antidote est à déterminer de façon individuelle pour chaque patient et chaque traitement.

La présente invention vise, par conséquent, à fournir un appareil permettant de pratiquer les hémodialyses sans avoir recours à des anticoagulants en commandant un rinçage périodique des membranes du dialyseur au moyen d'un liquide physiologique.

Le but de la présente invention est atteint par un appareil pour la pratique de l'hémodialyse sans anticoagulants, caractérisé en ce qu'il comporte un hémodialyseur dont le tuyau d'entrée sur lequel est montée une pompe volumétrique est la réunion d'un conduit d'amenée de sang prélevé au patient et d'un conduit d'alimentation de liquide physiologique, chacun de ces conduits comportant un organe de contrôle de passage commandé pour ouvrir et fermer alternativement chaque conduit.

Suivant un mode d'exécution particulièrement avantageux de la présente invention, la réunion du conduit d'amenée de sang prélevé au patient et du conduit d'alimentation de liquide physiologique est réalisée au niveau du moniteur de pression à deux entrées.

De préférence, les organes de contrôle de passage montés sur le conduit d'amenée de sang et sur le conduit d'alimentation de liquide physiologique sont des clamps ou tout autre moyen de fermeture par écrasement du conduit.

De cette manière, les conduits restent connectés et une bonne stérilité est maintenue.

Par ailleurs, les clamps et le dispositif de commande de ceux-ci peuvent être montés dans un boîtier séparé de telle sorte que la commande de liquide de rinçage peut être branchée en cours d'opération d'hémodialyse sans interruption de celle-ci, étant donné que tout conduit préalablement stérilisé n'est pas déconnecté pour le branchement, mais que toute manipulation se fait à l'extérieur des conduits.

Suivant l'invention, le dispositif de commande destiné à ouvrir et fermer alternativement chaque conduit au moyen des organes de contrôle de passage de telle sorte que lorsque l'un des conduits est ouvert, l'autre est fermé, consiste avantageusement en des circuits à temporisation réglable.

L'appareil selon l'invention est décrit plus en détail à l'appui des figures annexées, dans lesquelles :

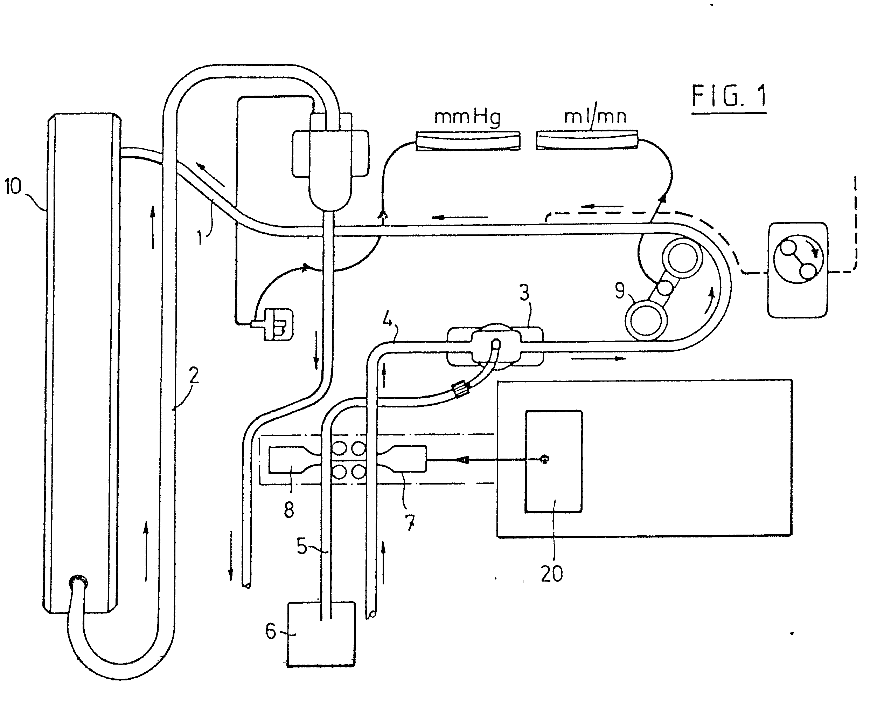

La figure 1 est un schéma synoptique de l'appareil selon l'invention,

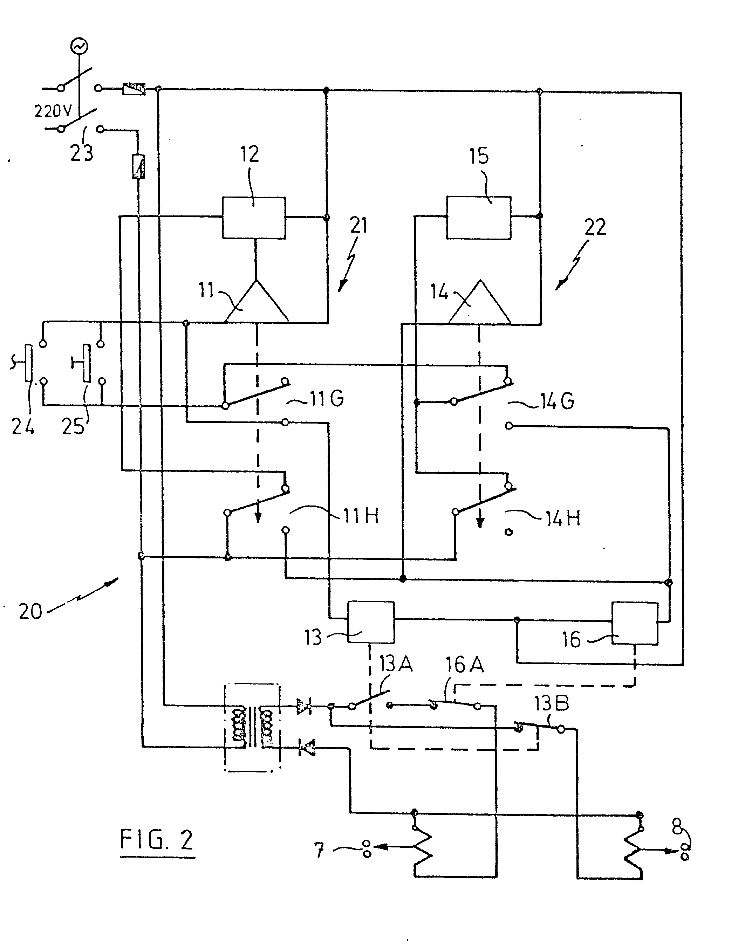

La figure 2 est un schéma électrique simplifié du dispositif de commande faisant partie de l'appareil.

L'appareil comprend un dialyseur 10 connu en soi, ayant une entrée connectée à un tuyau 1 pour recevoir le sang prélevé au patient et ayant une sortie connectée à un tuyau 2 qui renvoie le sang traité par le patient. Le tuyau d'entrée est connecté à la sortie d'un moniteur de pression artérielle 3 ayant deux entrées : une entrée connectée au conduit 4 d'amenée de sang et une entrée connectée à un tuyau 5 qui achemine un liquide physiologique de rinçage, liquide qui contient 9 g/l de NaCl, à partir d'un flacon 6. Le passage du sang dans le tuyau 4 et le passage du liquide physiologique dans le tuyau 5 sont contrôlés respectivement par le clamp 7 et par le clamp 8, eux-mêmes commandés par un dispositif de commande 20. La circulation du sang ou du liquide physiologique vers le dialyseur 10 est assurée par une pompe à débit constant 9.

Le dispositif de commande 20 est organisé pour envoyer à une fréquence prédéterminée une quantité précise et reproductive de liquide physiologique pour rincer la membrane du dialyseur et les lignes artério-veineuses, le débit de sang étant interrompu pendant les opérations de rinçage. Le dispositif de commande 20 comprend deux circuits à temporisation 21 et 22, l'un servant à la commande du clamp artériel 7 et l'autre à la commande du clamp de liquide physiologique 8. Le circuit 21 comprend un électro-aimant 11 couplé à un moteur synchrone 12 et un relais à deux contacts 13. Le circuit 22 comprend un électro-aimant 14 couplé à un moteur synchrone 15 et un relais 16 à un contact. Chacun des électro-aimants 11 et 14 a deux contacts : un contact instantané G et un contact temporisé H. les électro-aimants 11 et 14 et les relais 13 et 16 sont interconnectés de telle manière que la fermeture du contact temporisé H de l'un quelconque des deux circuits 21 et 22 provoque l'excitation de l'électro-aimant de l'autre circuit, ce qui produit l'ouverture alternée des deux clamps 7 et 8 à la cadence fixée par les valeurs des temporisations respectives dans les deux circuits.

Lors de la mise sous tension de l'appareil grâce à la fermeture de l'interrupteur général 23, l'électro-aimant 11 se trouve excité et ferme instantanément son contact 11G. Le contact du bouton de commande 24 sert à donner l'impulsion de départ pour la mise en fonctionnement du dispositif et le commutateur 25 en position fermée sert à commander le fonctionnement cyclique de l'appareil. Le relais 13 est alors excité et son contact 13A ferme le circuit d'excitation du clamp artériel 7. Après écoulement du délai de temporisation T1 fixé pour la période de passage du sang, par exemple 5 à 10 minutes, le moteur 12 provoque la fermeture du contact 11G. L'électro-aimant 14 se trouve alors excité et ferme son contact 14G, excitant ainsi le relais 16. Le contact 16A s'ouvre alors et interrompt le circuit d'excitation du clamp artériel 7 qui se ferme. En même temps, l'excitation de l'électro-aimant 11 a été interrompue et le contact 11G s'est ouvert, provoquant ainsi l'ouverture du contact 13A et la fermeture du contact 13B de manière à fermer le circuit d'excitation du clamp de liquide physiologique 8. Celui-ci s'ouvre pour laisser passer le liquide physiologique vers le dialyseur 10.

Après écoulement du délai de temporisation T2 fixé pour la période de passage du liquide physiologique, par exemple 10 à 15 secondes, le moteur 15 provoque la fermeture du contact 14H, ce qui a pour effet d'exciter à nouveau l'électro-aimant 11 et le cycle décrit plus haut se répète.

Il faut noter que, dans la construction avantageuse de l'appareil de l'invention comme décrit, la même pompe volumétrique 9 assure tant la circulation du sang prélevé du patient que celle du liquide physiologique.

Il est bien entendu que l'invention permet plusieurs formes d'exécution couvertes par la revendication 1 mais qui doivent toutes être conçues de manière judicieuse pour assurer la stérilité des circuits. De même, les circuits de commande et de temporisation réglables peuvent être des circuits logiques à semi-conducteurs.

Revendications

1. Appareil pour la pratique de l'hémodialyse sans anticoagulants, caractérisé en ce qu'il comporte un hémodialyseur (10) dont le tuyau d'entrée (1) sur lequel est montée une pompe volumétrique (9) est la réunion d'un conduit d'amenée (4) de sang prélevé au patient et d'un conduit d'alimentation (5) de liquide physiologique, chacun de ces conduits (4, 5) comportant un organe de contrôle de passage (7, 8) commandé pour ouvrir et fermer alternativement chaque conduit (4, 5).

2. Appareil suivant la revendication 1 caractérisé en ce que la réunion du conduit d'amenée (4) de sang prélevé au patient et du conduit d'alimentation (5) de liquide physiologique est effectuée au niveau du moniteur de pression à deux entrées.

3. Appareil suivant l'une des revendications 1 ou 2 caractérisé en ce que les organes de contrôle de passage (7, 8) montés sur le conduit d'amenée de sang (4) et sur le conduit d'alimentation (5) en liquide physiologique sont des clamps ou tout autre moyen de fermeture par écrasement du conduit.

4. Appareil suivant l'une quelconque des revendications précédentes, caractérisé en ce que le dispositif de commande comprend deux circuits à temporisation (21, 22) réglables connectés chacun pour produire un signal de commande pour un des organes de contrôle de passage (7, 8), lesdits circuits à temporisation comprenant chacun des moyens de commutation (11, 14) interconnectés en sorte que lorsque l'un des circuits produit un signal de commande de fermeture, l'autre circuit produit un signal de commande d'ouverture et en sorte que l'action temporisée de l'un quelconque des circuits déclenche l'action de l'autre circuit.

5. Appareil selon l'une quelconque des revendications précédentes, caractérisé en ce que les circuits à temporisation (21, 22) du dispositif de commande (20) comprennent chacun un électro-aimant (11, 14) ayant un contact à action instantanée (G) et un contact temporisé (H), le contact temporisé (11H) au repos étant inséré dans le circuit d'excitation du moteur (12) et le contact temporisé (11H) au travail étant inséré dans le circuit d'excitation de l'électro-aimant (14) tandis que le circuit d'excitation de l'électro-aimant (11) contient en série les contacts (14G et 14H) au repos.

6. Appareil suivant l'une quelconque des revendications précédentes, caractérisé en ce que les clamps (7, 8) et le dispositif de commande de ceux-ci sont montés dans un boîtier séparé.

7. Utilisation de l'appareil suivant l'une quelconque des revendications précédentes pour le traitement extracorporel du sang par hémodialyse.

FIG. 1

FIG. 2

Office européen
des brevets

**RAPPORT DE RECHERCHE EUROPEENNE**

EP  83 87 0054

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | FR-A-2 336 961  (RHONE-POULENC) <br> * En entier, en particulier figure 2, page 4, lignes 24-34 * <br><br> --- | 1,3,7 | A 61 M    1/03 |
| A | DE-A-2 825 134  (CORDIS) <br> * Page 23, ligne 15 - page 24, ligne 16 * <br><br> --- | 2 | |
| A | US-A-3 802 432  (DJERASSI) <br> * En entier, en particulier figure 2, signes de référence SW5,SW6,78,85, colonne 6, lignes 3-8 * <br><br> --- | 4,5 | |
| A | FR-A-2 310 136  (BERNAS) <br> * Figures 1,2 * <br><br> ----- | 6 | |

**DOMAINES TECHNIQUES RECHERCHES (Int. Cl. ³)**

A 61 M

Le présent rapport de recherche a été établi pour toutes les revendications

| Lieu de la recherche <br> LA HAYE | Date d'achèvement de la recherche <br> 12-09-1983 | Examinateur <br> PETZUCH M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82